# EUROPEAN PATENT SPECIFICATION

(11) **EP 0 541 662 B1**
(45) Date of publication and mention of the grant of the patent: **02.11.1994**
(21) Application number: 91914235.6
(22) Date of filing: 26.07.1991
(51) Int. Cl.: C07K 7/06, C07K 7/64, A61K 37/02, A61K 37/42

(54) **SYNTHETIC PEPTIDES THAT ANTAGONIZE NEUROKININ A, THEIR SALTS AND THEIR MANUFACTURING PROCESSES**
SYNTHETISCHE PEPTIDE MIT NEUROKININ A ANTAGONISTISCHER WIRKUNG, DEREN SALZE UND VERFAHREN ZU DEREN HERSTELLUNG
PEPTIDES DE SYNTHESE ANTAGONISTES DE LA NEUROKININE A, LEURS SELS ET LEURS PROCEDES DE FABRICATION

(30) Priority: 03.08.1990 IT 945590
(43) Date of publication of application: 19.05.1993
(73) Proprietor: A. MENARINI INDUSTRIE FARMACEUTICHE RIUNITE S.R.L., I-50131 Firenze (IT)
(72) Inventor: GIULIANI, Sandro, I-50012 Bagno a Ripoli, Firenze (IT); MAGGI, Carlo, Alberto, I-50141 Firenze (IT); PATACCHINI, Riccardo, I-50144 Firenze (IT); PESTELLINI, Vittorio, I-50129 Firenze (IT); QUARTARA, Laura, I-52037 Sansepolcro, Arezzo (IT); ROVERO, Paolo, I-50145 Firenze (IT)
(74) Representative: Mannucci, Gianfranco, Dott.-Ing.
(86) International application number: IT9100067
(87) International publication number: WO9202546

(56) References cited:
- EP-A- 401 177

## Description

### FIELD OF THE INVENTION

The invention relates to linear and cyclic synthetic peptides which antagonize neurokinin A.

The invention further relates to salts of said peptides, and to the processes for obtaining said peptides and their respective salts.

The abbreviations used are listed at the end of the present description.

### BACKGROUND OF THE INVENTION

Tachykinins are a group of peptides that have the same C-terminal sequence:
Phe-X-Gly-Leu-Met-NH₂.

At least three peptides belonging to this group have been identified in mammals, namely substance P (SP), neurokinin A (NKA) and neurokinin B (NKB). These have been shown to act as neurotransmitters both peripherally and centrally. Cf.: J.E. Maggio, Peptides 1985, 6: 237-245, and P.C. Emson et al. in Neuropeptides and their peptidases, A.J. Turner, ed. Ellis Horwood, England, 1987, pp. 87-106.

The pharmacological and biochemical results reported in the literature show that the biological activity of the tachykinins is mediated in mammal tissue by at least three separate receptors, known as NK-1, NK-2 and NK-3. Cf.: S.H. Buck et al., Science 1984, 226: 987-989; R. Laufer et al., Proc. Natl. Acad. Sci. USA 1985, 82: 7444-7449 and C.M. Lee et al., Eur. J. Pharmacol. 1986, 130: 209-216. Natural tachykinins show varying affinity for these three receptors: SP, NKA and NKB are the most powerful agonists of the receptors NK-1, NK-2 and NK-3 respectively. However, in high concentrations the three natural tachykinins can stimulate each of the three receptors to an equal degree by virtue of their common C-terminal sequence. The development of antagonists capable of selectively blocking one of the three receptors for the tachykinins therefore seems to be of major importance. The first generation of tachykinin antagonists, as described, for example, in U.S. Patent No. 4,481,139, in fact showed little selectivity (S.H. Buck & S.A. Shatzer, Life Sci. 1988, 42: 2701-2708). By making amino acid substitutions of the same type but in the C-terminal sequence of NKA, which is per se more selective than SP, we have already disclosed (cf. Italian Patent No. 9432 A/89, EP-A-0 401 177) second-generation peptide antagonists with good selectivity for the receptor NK2.

### SUMMARY OF THE INVENTION

The subject of the present invention is linear and cyclic synthetic peptides with the general formula:

X-Asp-Tyr-DTrp-Val-DTrp-DTrp-Y I

where: Y = A-R;
A is one of the following amino acids:
Lys, Orn, Glu, Gln, Asp, Asn, His, Ala, βAla, Val, Leu, Nle, Ile, Gly, Pro, Trp, Ser, Thr;
R = NH₂ (C-terminal carboxamide);
X = H (free N-terminal amino group)
or
A is one of the following amino acids:
Arg, Lys, Orn, Glu, Gln, Asp, Asn, His, Ala, βAla, Val, Leu, Nle, Ile, Gly, Pro, Phe, Trp, DTrp, Tyr, Met, Ser, Thr;
when R and X together constitute an amide bond that joins together the two ends of the peptide chain to form a cyclic peptide;
and their pharmaceutically acceptable salts with organic and inorganic acids or bases. These peptides exhibit competitive antagonism of neurokinin A and also have a high degree of selectivity for the receptor NK-2.

The following in particular also form part of the invention:
- a linear heptapeptide with the following structure:
   H-Asp-Tyr-DTrp-Val-DTrp-DTrp-Lys-NH₂;
- a linear heptapeptide with the following structure:
   H-Asp-Tyr-DTrp-Val-DTrp-DTrp-Ala-NH₂;
- a linear heptapeptide with the following structure:
   H-Asp-Tyr-DTrp-Val-DTrp-DTrp-Orn-NH₂;
- a linear heptapeptide with the following structure:
   H-Asp-Tyr-DTrp-Val-DTrp-DTrp-Glu-NH₂;
- a linear heptapeptide with the following structure:
   H-Asp-Tyr-DTrp-Val-DTrp-DTrp-Leu-NH₂;
- a linear heptapeptide with the following structure:
   H-Asp-Tyr-DTrp-Val-DTrp-DTrp-Nle-NH₂;
- a linear heptapeptide with the following structure:
   H-Asp-Tyr-DTrp-Val-DTrp-DTrp-Gly-NH₂;
- a cyclic heptapeptide with the following structure:
   cyclo[AspTyrDTrpValDTrpDTrpArg];
- a cyclic heptapeptide with the following structure:
   cyclo[AspTyrDTrpValDTrpDTrpLys].

The new peptides to which the present application relates can reduce or antagonize the pathological effects of an excess of neurokinin A; they are therefore useful in the treatment of arthritis, asthma, inflammation, tumour growth, gastrointestinal hypermotility, Huntington's chorea, neuritis, neuralgia, migraine, hypertension, urinary incontinence, urticaria, symptoms of the carcinoid syndrome, influenza and the common cold.

The linear and cyclic peptides that are the subject of the present invention can be prepared by the known methods of peptide synthesis both in solution and in solid phase, as described, for example, in: M. Bodanszky & A. Bodanszky, The Practice of Peptide Synthesis, Springer-Verlag, Berlin Heidelberg, 1984., J. Stewart & J. Young, Solid Phase Peptide Synthesis, Pierce Chemical Co., Rockford, 1984; E. Atherton & R.C. Sheppard, Solid Phase Peptide Synthesis, IRL Press, Oxford, 1989. By way of a non-restrictive example we describe the synthesis process in solid phase for linear peptides and the synthesis process and cyclization in solid phase for cyclic peptides.

To obtain linear peptides with the C-terminal carboxyl group in the form of carboxamide by the method in solid phase, one can use solid supports such as the resin 4-methylbenzhydrylamine (MBHA) or the "resin for peptides in amide form with Fmoc strategy" (Dod), described by J. Knolle et al., Tetrahedron Lett., 1987, 28: 5651. In the first case, the alpha-amino function of the amino acids is protected by the t-butyloxycarbonyl group (Boc), which can be selectively deprotected with trifluoroacetic acid, while the final deprotection, with simultaneous detachment of the peptide in the amide form from the support polymer, is achieved with anhydrous hydrofluoric acid. In the case of the Dod resin, the alpha-amine function of the amino acids is protected by the 9-fluorenyl-methoxycarbonyl group (Fmoc), which can be deprotected selectively with piperidine, while the final deprotection, with simultaneous detachment of the peptide in the amide form from the support polymer, is achieved with trifluoroacetic acid. In both cases the side chains of the trifunctional amino acids can be protected by the known methods described in the literature. To construct the peptide chain on the insoluble polymer support, each amino acid is reacted as it is, i.e. in the form of the free acid, in the presence of a suitable coupling agent such as dicyclohexylcarbodiimide (DCC), if necessary with the addition of suitable additives, such as hydroxybenzotriazole (HOBt) or benzotriazolyl-N-oxytrisdimethylaminophosphonium hexafluorophosphate (BOP); alternatively, the amino acid can react in the form of the symmetrical anhydride, active ester or by one of the other methods described in the literature. The completeness of the amino acid coupling reactions can be checked by the ninhydrin test as described by E.T. Kaier et al., Anal. Biochem., 1970, 34: 595. After the crude peptide, suitably freeze-dried, has been detached from the resin, it is purified to render it homogeneous, for example by chromatographic methods such as high pressure preparative chromatography in inverse phase.

To synthesize the cyclic peptides the method of cyclization in solution can be used, with the linear precursor of the desired cyclic peptide first being prepared by one of the methods described above, either in solution or in solid phase. The cyclization is achieved with the aid of suitable condensing agents and, if necessary, by activating the C-terminal carboxyl group of the linear precursor, for example in the form of azide. Alternatively, a method of cyclization in solid phase can conveniently be used, by virtue of the presence in the sequence to be synthesized of a residue of aspartic acid which can be anchored covalently to an appropriate resin, for example phenylacetamidomethyl resin (PAM), by the carboxyl function in the beta position. Subsequently, when the carboxyl function in the alpha position has been protected in the form of fluorenylmethyl ester, the elongation of the chain can be achieved by the normal Boc strategy outlined above for the synthesis of the linear peptides. Having obtained the linear precursor, again coupled to the resin by the side chain of aspartic acid, it is possible to deprotect selectively the carboxyl group in the alpha position of the aspartic acid itself and bring about the cyclization by intramolecular condensation between the carboxyl group in the alpha position of the aspartic acid and the amino group in the alpha position of the amino acid at the N-terminal end of the same chain. This condensation is conveniently achieved in the presence of benzotriazolyl-N-oxytrisdimethylaminophosphonium hexafluorophosphate (BOP). The cyclic peptide is then deprotected and detached from the solid support with the aid of anhydrous hydrofluoric acid. The crude cyclic peptide thus obtained can easily be purified by high-pressure preparative chromatographic methods in reverse phase.

### DETAILED DESCRIPTION OF PREFERRED EMBODIMENTS

### Example 1

Synthesis of the linear peptide with the following sequence:
H-Asp-Tyr-DTrp-Val-DTrp-DTrp-Lys-NH₂.

1.0 g of DOD resin (Bachem, Switzerland), equivalent to 0.50 mmol of amino groups, is placed in the reaction chamber of a Labortec SP 640 semi-automatic peptide synthesizer. The resin is then washed in accordance with the protocol set out in Table 1 below, cycles 7-8, then it is deprotected (cycles 1-3) and washed again (cycles 4-8). To couple the N-terminal amino acid to the resin, a solution of HOBt (0.306 g) and Fmoc-Lys(Boc)-OH (0.702 g) in 10 ml DCM/DMF (2/1 v/v) is added to the deprotected resin. The resin is stirred for 2 min (cycle 9), then a 1M solution of DCC (1.5 ml) in DCM/DMF (2/1 v/v) is added and stirring continued at ambient temperature for a further 60 min (cycle 10). The washing procedures are then carried out (cycles 11-14) and the reaction is checked by the ninhydrin test as described by Kaiser. If the result is negative, the Fmoc group is hydrolyzed with PIP (cycles 1-8) before proceeding with the coupling of the next amino acid, which is effected in the same way. The following residues are reacted in the order and the quantities given: Fmoc-DTrp-OH (0.639 g), Fmoc-DTrp-OH (0.639 g), Fmoc-Val-OH (0.509 g), Fmoc-DTrp-OH (0.639 g), Fmoc-Tyr(tBu)-OH (0.689 g) and Fmoc-Asp(OtBu)-OH (0.617 g). After the last coupling, the N-terminal amino group of the peptide anchored to the resin was deprotected (cycles 1-3) and the peptide washed (cycles 4-8) and dried under vacuum, yielding 1.60 g dry product. For the final deprotection, the peptide anchored to the resin was placed in a 250 ml flask containing 27 ml TFA, 1.5 ml p-cresol and 1.5 ml ethanedithiol. The resulting suspension is stirred at 37°C for 2 h, then the resin is filtered off and washed with 3 batches of 5 ml TFA. The resulting solution is diluted with 250 ml cold ethyl ether plus 150 ml cold petroleum ether, which causes a white solid to precipitate out. The suspension is cooled to -78°C and the crude peptide is filtered, washed three times with cold ether, dissolved in water and freeze-dried to give 0.375 g crude product. The peptide is finally purified by liquid chromatography in reverse phase and analyzed by analytical hplc on a Waters C₁₈ Deltapak column, 3.9 × 150 mm, with a gradient of acetonitrile containing 0.1% (v/v) trifluoroacetic acid (phase B) against 0.1% (v/v) aqueous trifluoroacetic acid (phase A), between 20 and 80% of B in 20 minutes at a flow rate of 1 ml/minute, with UV detection at 210 nm. Retention time (Rt) = 9.6 minutes; chromatographic purity ≧ 99%.

### Example 2

Synthesis of the linear peptide with the following sequence:
H-Asp-Tyr-DTrp-Val-DTrp-DTrp-Ala-NH₂.

1.0 g of MBHA resin (Novabiochem, Switzerland), equivalent to 0.45 mmol of amino groups, is placed in the reaction chamber of a Labortec SP 640 semi-automatic peptide synthesizer. The resin is neutralized with Et₃N in a 10% solution (v/v) in CHCl₃ (2 × 15 ml; 5 + 15 min) and washed repeatedly with DCM. The coupling of Boc-Ala-OH is then effected by the method of the preformed symmetrical anhydride: 0.340 g of the protected amino acid is dissolved in 3 ml DCM and 2.32 ml of an 8% (wt/v) solution of DCC in DCM are added, with magnetic stirring on an ice-bath being continued for 15 min. The mixture is then filtered through a funnel with a porous baffle to eliminate the DCU formed and the solution added to the reaction chamber of the synthesizer, diluting with a further 5 ml DMF and then commencing the automatic cycle of coupling and washing described in Table 2 below (cycles 8-12). The Kaiser test is then performed on an aliquot of resin; if the result is negative, the automatic deprotection of the amino group is effected (cycles 1-7). For subsequent couplings, again according to the protocol in Table 2, the following amino acids were used in the order and the quantities given: Boc-DTrp-OH (0.545 g), Boc-DTrp-OH (0.545 g), Boc-Val-OH (0.390 g), Boc-DTrp-OH (0.545 g), Boc-Tyr(2-BrZ)-OH (0.890 g) and Boc-Asp(OcHx)-OH (0.560 g). After the final coupling, the N-terminal amino group is deprotected (cycles 1-7), and the resin is removed from the reaction chamber and dried over potash under vacuum, giving 1.5 g of product. The peptide is then detached from the resin and the side chains deprotected with anhydrous hydrofluoric acid. The peptide anchored to the resin is placed in a "Teflon" reactor with 1.5 ml anisole and 0.15 ml dimethyl sulfide; this mixture is then cooled to -50°C and 15 ml anhydrous hydrofluoric acid are distilled off, then magnetic stirring is maintained on an ice bath for 60 min. The hydrofluoric acid is removed with a puff of nitrogen, the crude product is dried under aspiration for approximately 2 h, washed with ethyl ether (2 × 15 ml) and extracted in 50% acetic acid (3 x 15 ml), filtering through a funnel with a porous baffle to remove the exhausted resin. The resulting solution is diluted with water and freeze-dried to give 0.250 g crude product. The peptide is finally purified by liquid chromatography in reverse phase and analyzed by analytical hplc under the conditions described in the preceding example: Rt = 11.1 min; chromatographic purity ≧ 99%.

### Example 3

Synthesis of the cyclic peptide with the following sequence:
Cycle [Asp-Tyr-DTrp-Val-DTrp-DTrp-Arg].

2 g of the above-mentioned "PAM" resin (Bachem, Switzerland) bearing esterified aspartic acid on the benzyl linker of the resin by virtue of the beta-carboxyl function of the acid (degree of substitution: 0.35 mmol/g) are placed in the reaction chamber of a manual synthesizer. The aspartic acid is also protected on the alpha-amino function by Boc and on the alpha-carboxyl function in the form of a fluorenylmethyl ester. After repeated washing of the resin with DCM and DMF, the alpha-amino group of the aspartic acid is deprotected with TFA under the conditions normally used in the Boc strategy as described in the preceding example. The synthesis then proceeds in accordance with the standard Boc protocol (see Example 2); the following amino acids are used in the order and the quantities given: Boc-Arg(Tos)-OH (0.900 g), Boc-DTrp-OH (0.638 g), Boc-DTrp-OH (0.638 g), Boc-Val-OH (0.456 g), Boc-DTrp-OH (0.638 g) and Boc-Tyr(2-BrZ)-OH (1.038 g). Each coupling is repeated twice before proceeding with the deprotection with TFA. Once the protected linear precursor has been assembled, the N-terminal amino group is deprotected with TFA and then the C-terminal carboxyl group with PIP. The cyclization is achieved by treating the linear precursor anchored to the resin in a solution of DMF with BOP (0.929 g) and DIEA (0.731 ml) for three hours at ambient temperature. The process is repeated twice until the Kaiser test gives a negative result. Finally, the cyclized peptide is detached from the resin and, at the same time, the side chains are deprotected with anhydrous hydrofluoric acid, by the procedure described in Example 2, to give 0.545 g crude product. The peptide is purified by liquid chromatography in reverse phase and analyzed by analytical hplc under the conditions described in the preceding example:
Rt = 13.1 min, chromatographic purity ≧ 99%.

Further, non-restrictive examples of compounds according to the general formula I that are included in the following invention are:
H-Asp-Tyr-DTrp-Val-DTrp-DTrp-Glu-NH₂, Rt = 10.5 min;
H-Asp-Try-DTrp-Val-DTrp DTrp-Leu-NH₂, Rt = 12.2 min;
H-Asp-Tyr-DTrp-Val-DTrp-DTrp-Orn-NH₂, Rt = 8.9 min;
H-Asp-Tyr-DTrp-Val-DTrp-DTrp-Nle-NH₂, Rt = 12.2 min;
H-Asp-Tyr-DTrp-Val-DTrp-DTrp-Gly-NH₂, Rt = 10.5 min;
cyclo[AspTyrDTrpValDTrpDTrpLys], Rt = 10.8 min;
where Rt is the retention time in analytical HPLC under the conditions described in Example 1.

### BIOLOGICAL ACTIVITY

The ability of the peptides described in the present invention to interact with the neurokinin A receptor as agonists or antagonists was evaluated in an in-vitro study using a preparation in which the biological response produced by tachykinins and related peptides is determined exclusively from the neurokinin A receptor (NK-2 receptor). This preparation is isolated rabbit pulmonary artery, in which tachykinins produce dose-related contraction (Rovero et al., Neuropeptides, 1989, 13: 263-270). The activity of the peptides in this preparation was determined using a concentration of NKA (3 nM) that produces a response equivalent to 45% of the maximum. The peptides that are the subject of the present application were added to the preparation in increasing concentrations and their activity evaluated in terms of inhibition of the response to NKA. Table 3 below shows the results obtained with a 10⁻⁶M concentration of each compound.

Tables 1, 2 and 3 referred to above follow.

**Table 1**

| Protocol for automatic synthesis according to the Fmoc strategy | | |
|---|---|---|
| Cycle | Reagent | Time |
| 1 | DMF | 1 × 1 min |
| 2 | 20 % PIP / DMF | 1 × 3 min |
| 3 | 20 % PIP / DMF | 1 × 7 min |
| 4 | DCM | 2 × 1 min |
| 5 | DMF | 2 × 1 min |
| 6 | iPrOH | 2 × 1 min |
| 7 | DMF | 3 × 1 min |
| 8 | DCM | 3 × 1 min |
| 9 | Fmoc-AA + HOBt / DMF /DCM | 1 × 2 min |
| 10 | DCC 1 M DMF / DCM | 1 × 90 min |
| 11 | iPrOH | 1 × 1 min |
| 12 | DMF | 1 × 1 min |
| 13 | DCM | 1 × 1 min |
| 14 | Repeat twice from cycle 11 | |
| Volume of solvents: 10-20 ml/g resin | | |

**Table 2**

| Protocol for automatic synthesis according to the Boc strategy | | |
|---|---|---|
| Cycle | Reagent | Time |
| 1 | DCM | 1 × 1 min |
| 2 | 50 % TFA / DCM | 1 × 5 min |
| 3 | 50 % TFA / DCM | 1 × 15 min |
| 4 | DCM | 3 × 1 min |
| 5 | 5 % DIEA / DCM | 2 × 1 min |
| 6 | DCM | 2 × 1 min |
| 7 | DMF | 2 × 1 min |
| 8 | Boc-AA anhydride in DCM / DMF | 1 × 60 min |
| 9 | DMF | 1 × 1 min |
| 10 | DCM | 1 × 1 min |
| 11 | Repeat cycles 8 and 9 | |
| 12 | DCM | 3 × 1 min |
| Volume of solvents: 10-20 ml/g resin. | | |

Antagonist biological activity on the NK-2 receptor in isolated rabbit pulmonary artery of the peptides described.

| Peptide | % Inhibition |
|---|---|
| H-Asp-Tyr-DTrp-Val-DTrp-DTrp-Lys-NH₂ | 100 |
| H-Asp-Tyr-DTrp-Val-DTrp-DTrp-Ala-NH₂ | 100 |
| H-Asp-Tyr-DTrp-Val-DTrp-DTrp-Orn-NH₂ | 100 |
| H-Asp-Tyr-DTrp-Val-DTrp-DTrp-Glu-NH₂ | 100 |
| H-Asp-Tyr-DTrp-Val-DTrp-DTrp-Leu-NH₂ | 100 |
| H-Asp-Tyr-DTrp-Val-DTrp-DTrp-Nle-NH₂ | 100 |
| H-Asp-Tyr-DTrp-Val-DTrp-DTrp-Gly-NH₂ | 100 |
| Cyclo[AspTyrDTrpValDTrpDTrpLys] | 95 |
| Cyclo[AspTyrDTrpValDTrpDTrpArg] | 95 |

### List of abbreviations used

For the nomenclature and abbreviations of the amino acids please refer to the Recommendations of the IUPAC-IUB Joint Commission on Biochemical Nomenclature (Eur. J. Biochem., 1984, 138: 9); the amino acids are in the L configuration unless otherwise stated.

The other abbreviations used are: 2-Br-Z: 2-bromobenzyloxycarbonyl; AAA: amino acid analysis (on the hydrolyzed peptide); Boc: tert-butyloxycarbonyl; BOP: benzotriazolyl-N-oxytrisdimethylaminophosphonium hexafluorophosphate; Bzl: benzyl; DCC: N,N'-dicyclohexylcarbodiimide; DCM: dichloromethane; DCU: N,N'-dicyclohexylurea; DIEA: diisopropylethylamine; DMF: N,N-dimethylformamide; Et₃N: triethylamine; FAB-MS: fast atom bombardment mass spectrometry; Fmoc: 9-fluorenylmethoxycarbonyl; HOBt: 1-hydroxybenzotriazole; HPLC: high pressure liquid chromatography; iPrOH: isopropanol; MBHA: 4-methylbenzhydrylamine; NKA: neurokinin A; NKB: neurokinin B; OcHx: cyclohexyl ester; PIP: piperidine; SP: substance P; tBu: tert-butyl; TFA: trifluoroacetic acid, Tos: tosyl(4-toluenesulfonyl).

## Claims

1. A linear or cyclic synthetic peptide with the general formula:
X-Asp-Tyr-DTrp-Val-DTrp-DTrp-Y I
where: Y = A-R;
A is one of the following amino acids:
Lys, Orn, Glu, Gln, Asp, Asn, His, Ala, βAla, Val, Leu, Nle, Ile, Gly, Pro, Trp, Ser, Thr;
R = NH₂ (C-terminal carboxamide);
X = H (free N-terminal amino group)
or
A is one of the following amino acids:
Arg, Lys, Orn, Glu, Gln, Asp, Asn, His, Ala, βAla, Val, Leu, Nle, Ile, Gly, Pro, Phe, Trp, DTrp, Tyr, Met, Ser, Thr;
when R and X together constitute an amide bond that joins together the two ends of the peptide chain to give a cyclic peptide;
and its pharmaceutically acceptable salts with organic and inorganic acids or bases.

2. A synthetic heptapeptide as claimed in claim 1 with the following structure:
H-Asp-Tyr-DTrp-Val-DTrp-Dtrp-Lys-NH₂;
and its pharmaceutically acceptable salts with organic and inorganic acids or bases.

3. A synthetic heptapeptide as claimed in claim 1 with the following structure:
H-Asp-Tyr-DTrp-Val-DTrp-DTrp-Ala-NH₂,
and its pharmaceutically acceptable salts with organic and inorganic acids or bases.

4. A synthetic heptapeptide as claimed in claim 1 with the following structure:
H-Asp-Tyr-DTrp-Val-DTrp-DTrp-Orn-NH₂,
and its pharmaceutically acceptable salts with organic and inorganic acids or bases.

5. A synthetic heptapeptide as claimed in claim 1 with the following structure:
H-Asp-Tyr-DTrp-Val-DTrp-DTrp-Glu-NH₂,
and its pharmaceutically acceptable salts with organic and inorganic acids or bases.

6. A synthetic heptapeptide as claimed in claim 1 with the following structure:
H-Asp-Tyr-DTrp-Val-DTrp-DTrp-Leu-NH₂,
and its pharmaceutically acceptable salts with organic and inorganic acids or bases.

7. A synthetic heptapeptide as claimed in claim 1 with the following structure:
H-Asp-Tyr-DTrp-Val-DTrp-DTrp-Nle-NH₂,
and its pharmaceutically acceptable salts with organic and inorganic acids or bases.

8. A synthetic heptapeptide as claimed in claim 1 with the following structure:
H-Asp-Tyr-DTrp-Val-DTrp-DTrp-Gly-NH₂,
and its pharmaceutically acceptable salts with organic and inorganic acids or bases.

9. A synthetic cyclic heptapeptide as claimed in claim 1 with the following structure:
cyclo[AspTyrDTrpValDTrpDTrpArg]
and its pharmaceutically acceptable salts with organic and inorganic acids or bases.

10. A synthetic cyclic heptapeptide as claimed in claim 1 with the following structure:
cyclo[AspTyrDTrpValDTrpDTrpLys]
and its pharmaceutically acceptable salts with organic and inorganic acids or bases.

11. The use of a peptide as claimed in claim 1 to prepare a pharmaceutical composition for the treatment of asthma, inflammation and/or arthritis.

12. A process for obtaining peptides as claimed in claim 1 in which:
- synthesis is effected in solid phase with elongation of the peptide chain from the C-terminal end towards the N-terminal end on an insoluble polymer support;
- the subsequent amino acid, suitably protected both on the alpha-amine function and on the side chain, is reacted in activated form on the C-terminal amino acid, suitably protected on the side chain and anchored to the polymer support by the carboxyl function, in polar organic solvents;
- the subsequent couplings are effected using the appropriate amino acid, suitably protected, again in activated form, in the same manner;
- the peptide is then detached.

13. The process as claimed in claim 12 in which, to obtain a linear peptide, the peptide is detached from the support at the end and suitably deprotected.

14. The process as claimed in claim 12 in which, to obtain a cyclic peptide, the peptide is detached from the support in a suitable milieu and then cyclized using suitable activating and condensing agents, and the protecting groups are then appropriately removed.

15. The process as claimed in claim 12 in which, to obtain cyclic peptides containing at least one amino acid with two carboxylic residues, when the carboxylic residue is not in the alpha position but is attached to the resin, the carboxyl in the alpha position is deprotected then cyclized using the appropriate activating and condensing agents and the peptide is then detached from the support and the protecting groups removed.

## Patentansprüche

1. Lineares oder cyclisches, synthetisches Peptid der allgemeinen Formel
X-Asp-Tyr-DTrp-Val-DTrp-DTrp-Y I
in der
Y = A - R bedeutet, wobei
A eine der folgenden Aminosäuren:
Lys, Orn, Glu, Gln, Asp, Asn, His, Ala, βAla, Val, Leu, Nle, Ile, Gly, Pro, Trp, Ser, Thr;
R = NH₂ (C-terminales Carboxyamid) und
X = H (freie N-terminale Aminogruppe) ist
oder
A eine der folgenden Aminosäuren ist:
Arg, Lys, Orn, Glu, Gln, Asp, Asn, His, Ala, βAla, Val, Leu, Nle, Ile, Gly, Pro, Phe, Trp, DTrp, Tyr, Met, Ser, Thr;
wenn R und X zusammen eine Amidbindung bilden, die die zwei Enden der Peptidkette unter Ausbildung eines cyclischen Peptids verknüpft;
sowie seine pharmazeutisch verträglichen Salze mit organischen und anorganischen Säuren oder Basen.

2. Synthetisches Heptapeptid nach Anspruch 1 mit der folgenden Struktur:
H-Asp-Tyr-DTrp-Val-DTrp-DTrp-Lys-NH₂;
sowie seine pharmazeutisch verträglichen Salzen mit organischen und anorganischen Säuren oder Basen.

3. Synthetisches Heptapeptid nach Anspruch 1 mit der folgenden Struktur:
H-Asp-Tyr-DTrp-Val-DTrp-DTrp-Ala-NH₂,
sowie seine pharmazeutisch verträglichen Salze mit organischen und anorganischen Säuren oder Basen.

4. Synthetisches Heptapeptid nach Anspruch 1 mit der folgenden Struktur:
H-Asp-Tyr-DTrp-Val-DTrp-DTrp-Orn-NH₂,
sowie seine pharmazeutisch verträglichen Salze mit organischen und anorganischen Säuren oder Basen.

5. Synthetisches Heptapeptid nach Anspruch 1 mit der folgenden Struktur:
H-Asp-Tyr-DTrp-Val-DTrp-DTrp-Glu-NH₂,
sowie seine pharmazeutisch verträglichen Salze mit organischen und anorganischen Säuren oder Basen.

6. Synthetisches Heptapeptid nach Anspruch 1 mit der folgenden Struktur:
H-Asp-Tyr-DTrp-Val-DTrp-DTrp-Leu-NH₂,
sowie seine pharmazeutisch verträglichen Salze mit organischen und anorganischen Säuren oder Basen.

7. Synthetisches Heptapeptid nach Anspruch 1 mit der folgenden Struktur:
H-Asp-Tyr-DTrp-Val-DTrp-DTrp-Nle-NH₂,
sowie seine pharmazeutisch verträglichen Salze mit organischen und anorganischen Säuren oder Basen.

8. Synthetisches Heptapeptid nach Anspruch 1 mit der folgenden Struktur:
H-Asp-Tyr-DTrp-Val-DTrp-DTrp-Gly-NH₂,
sowie seine pharmazeutisch verträglichen Salze mit organischen und anorganischen Säuren oder Basen.

9. Synthetisches cyclisches Heptapeptid nach Anspruch 1 mit der folgenden Struktur:
cyclo[AspTyrDTrpValDTrpDTrpArg]
sowie seine pharmazeutisch verträglichen Salze mit organischen und anorganischen Säuren oder Basen.

10. Synthetisches cyclisches Heptapeptid nach Anspruch 1 mit der folgenden Struktur:
cyclo[AspTyrDTrpValDTrpDTrpLys]
sowie seine pharmazeutisch verträglichen Salze mit organischen und anorganischen Säuren oder Basen.

11. Verwendung eines Peptids nach Anspruch 1 zu Herstellung einer pharmazeutischen Zusammensetzung für die Behandlung von Asthma, Entzündungen und/oder Arthritis.

12. Verfahren zur Herstellung von Peptiden nach Anspruch 1, bei dem
- die Synthese in fester Phase unter Verlängerung der Peptidkette von dem C-terminalen Ende in Richtung auf das N-terminale Ende auf einem unlöslichen polymeren Träger durchgeführt wird;
- die nachfolgende Aminosäure, in geeigneter Weise geschützt sowohl an der alpha-Aminfunktion und an der Seitenkette, in einer aktivierten Form an der C-terminalen Aminosäure, in geeigneter Weise geschützt an der Seitenkette und verankert auf dem polymeren Träger über die Carboxylfunktion, in polaren organischen Lösemitteln umgesetzt wird;
- die anschließenden Kopplungen durchgeführt werden, indem man die geeignete Aminosäure, geeignet geschützt, wiederum in aktivierter Form, in der gleichen Weise verwendet, und
- das Peptid anschließend abgekoppelt wird.

13. Verfahren nach Anspruch 12, bei dem zum Erhalt eines linearen Peptids das Peptid von dem Träger am Ende abgekoppelt und in geeigneter Weise entschützt wird.

14. Verfahren nach Anspruch 12, bei dem zum Erhalt eines cyclischen Peptids das Peptid von dem Träger in einem geeigneten Medium abgekoppelt und anschließend unter Verwendung eines geeigneten Aktivierungs- und Kondensationsmittels cyclisiert wird, und schließlich die Schutzgruppen in geeigneter Weise entfernt werden.

15. Verfahren nach Anspruch 12, bei dem zum Erhalt von cyclischen Peptiden mit einem Gehalt von mindestens einer Aminosäure mit zwei Carboxylresten, wenn sich der Carboxylrest nicht in der alpha-Position befindet, sondern an das Harz gekoppelt ist, das Carboxyl in der alpha-Position entschützt und anschließend unter Verwendung der geeigneten Aktivierungs- und Kondensationsmittel cyclisiert wird, und anschließend das Peptid von dem Träger abgekoppelt und die Schutzgruppen entfernt werden.

## Revendications

1. Peptide synthétique linéaire ou cyclique ayant la formule générale:
X-Asp-Tyr-DTrp-Val-DTrp-DTrp-Y I
dans laquelle: Y = A-R;
A est l'un des aminoacides suivants:
Lys, Orn, Glu, Gln, Asp, Asn, His, Ala, βAla, Val, Leu, Nle, Ile, Gly, Pro, Trp, Ser, Thr;
R = NH₂ (carboxamide C-terminal);
X = H (groupe amino N-terminal libre)
ou
A est l'un des aminoacides suivants:
Arg, Lys, Orn, Glu, Gln, Asp, Asn, His, Ala, βAla, Val, Leu, Nle, Ile, Gly, Pro, Phe, Trp, DTrp, Tyr, Met, Ser, Thr;
lorsque R et X forment ensemble une liaison amide qui relie l'une à l'autre les deux extrémités de la chaîne peptidique pour donner un peptide cyclique; et ses sels pharmaceutiquement acceptables avec des acides ou des bases organiques ou inorganiques.

2. Heptapeptide synthétique selon la revendication 1, ayant la structure suivante:
H-Asp-Tyr-DTrp-Val-DTrp-DTrp-Lys-NH₂,
et ses sels pharmaceutiquement acceptables avec des acides ou des bases orgamques ou inorganiques.

3. Heptapeptide synthétique selon la revendication 1, ayant la structure suivante:
H-Asp-Tyr-DTrp-Val-DTrp-DTrp-Ala-NH₂,
et ses sels pharmaceutiquement acceptables avec des acides ou des bases organiques ou inorganiques.

4. Heptapeptide synthétique selon la revendication 1, ayant la structure suivante:
H-Asp-Tyr-DTrp-Val-DTrp-DTrp-Orn-NH₂,
et ses sels pharmaceutiquement acceptables avec des acides ou des bases organiques ou inorganiques.

5. Heptapeptide synthétique selon la revendication 1, ayant la structure suivante:
H-Asp-Tyr-DTrp-Val-DTrp-DTrp-Glu-NH₂,
et ses sels pharmaceutiquement acceptables avec des acides ou des bases organiques ou inorganiques.

6. Heptapeptide synthétique selon la revendication 1, ayant la structure suivante:
H-Asp-Tyr-DTrp-Val-DTrp-DTrp-Leu-NH₂,
et ses sels pharmaceutiquement acceptables avec des acides ou des bases organiques ou inorganiques.

7. Heptapeptide synthétique selon la revendication 1, ayant la structure suivante:
H-Asp-Tyr-DTrp-Val-DTrp-DTrp-Nle-NH₂,
et ses sels pharmaceutiquement acceptables avec des acides ou des bases organiques ou inorganiques.

8. Heptapeptide synthétique selon la revendication 1, ayant la structure suivante:
H-Asp-Tyr-DTrp-Val-DTrp-DTrp-Gly-NH₂,
et ses sels pharmaceutiquement acceptables avec des acides ou des bases organiques ou inorganiques.

9. Heptapeptide cyclique synthétique selon la revendication 1, ayant la structure suivante:
cyclo[AspTyrDTrpValDTrpDTrpArg],
et ses sels pharmaceutiquement acceptables avec des acides ou des bases organiques ou inorganiques.

10. Heptapeptide cyclique synthétique selon là revendication 1, ayant la structure suivante:
cyclo[AspTyrDTrpValDTrpDTrpLys],
et ses sels pharmaceutiquement acceptables avec des acides ou des bases organiques ou inorganiques.

11. Utilisation d'un peptide selon la revendication 1 pour la préparation d'une composition pharmaceutique pour le traitement de l'asthme, des inflammations et/ou de l'arthrite.

12. Procédé d'obtention de peptides selon la revendication 1, dans lequel:
- on effectue une synthèse en phase solide avec allongement de la chaîne peptidique de l'extrémité C-terminale vers l'extrémité N-terminale sur un support polymère insoluble;
- on fait réagir l'aminoacide suivant sous forme activée, convenablement protégé à la fois sur la fonction alpha-amino et sur la chaîne latérale, sur l'aminoacide C-terminal, convenablement protégé sur la chaîne latérale et ancré sur le support polymère par la fonction carboxyle, dans des solvants organiques polaires;
- on effectue de la même façon les couplages suivants avec l'aminoacide approprié, convenablement protégé, et encore sous forme activée;
- on détache ensuite le peptide.

13. Procédé selon la revendication 12, dans lequel, pour obtenir un peptide linéaire, on détache à la fin le peptide du support et on élimine de façon convenable les groupes protecteurs.

14. Procédé selon la revendication 12 dans lequel, pour obtenir un peptide cyclique, on détache le peptide du support dans un milieu convenable puis on le cyclise à l'aide d'agents d'activation et de condensation convenables, et on élimine ensuite les groupes protecteurs de façon appropriée.

15. Procédé selon la revendication 12, dans lequel, pour obtenir des peptides cycliques contenant au moins un aminoacide ayant deux résidus carboxyliques, lorsque le résidu carboxylique n'est pas en position alpha mais est fixé à la résine, on élimine le groupe protecteur du carboxyle en position alpha, puis on cyclise à l'aide des agents d'activation et de condensation appropriés, et on détache ensuite le peptide du support et on élimine les groupes protecteurs.
